# EUROPEAN PATENT APPLICATION

(11) **EP 1 508 324 A1**
(43) Date of publication of application: **23.02.2005**
(21) Application number: 04077188.3
(22) Date of filing: 30.07.2004
(51) Int. Cl.: A61K 7/16

(54) **Use of a zinc salt-containing oral care composition**

(30) Priority: 20.08.2003 GB 0319565
(71) Applicant: UNILEVER N.V., 3013 AL Rotterdam (NL); UNILEVER PLC, London EC4P 4BQ (GB)
(72) Inventor: Adams, Suzanne E., Unilever R & D Port Sunlight, Wirral Merseyside CH63 3JW (GB); Brading, Melanie G., Unilever R & D Port Sunlight, Wirral Merseyside CH63 3JW (GB); Cromwell, Victoria, Unilever R & D Port Sunlight, Wirral Merseyside CH63 3JW (GB); Green, Alison K., Unilever R & D Port Sunlight, Wirral Merseyside CH63 3JW (GB); Hall, Peter John, Unilever R & D Port Sunlight, Wirral Merseyside CH63 3JW (GB); Horay, Carmel P., Unilever R & D Port Sunlight, Wirral Merseyside CH63 3JW (GB)
(74) Representative: Newbould, Frazer Anthony

(57) **Abstract**

Use of a zinc salt-containing oral care composition for preventing or reducing acidification within dental plaque after consumption of a comestible, said comestible comprising a fermentable carbohydrate.

## Description

The present invention relates to the use of an oral care composition for preventing or reducing the acidification within dental plaque.

After eating foods it is not uncommon for the pH within dental plaque in the oral cavity to drop from around 7 to around 5.5. Further, this acidification can last for several hours. Continued and repeated acidification within dental plaque leads to irreversible damage of the tooth enamel and caries.

Surprisingly, the inventors have found that oral care compositions comprising zinc salts, in particular zinc citrate, can prevent or reduce the acidification that occurs within dental plaque after the consumption of a comestible. This is particularly so when the comestible comprises a fermentable carbohydrate.

An oral care composition capable of preventing the enamel demineralisation which follows continued and repeated acidification of the oral environment after eating such foods will thus be of benefit to the consumer.

Accordingly, the invention provides in a first aspect the use of a zinc salt-containing oral care composition for preventing or reducing acidification within dental plaque after consumption of a comestible, said comestible comprising a fermentable carbohydrate.

In a second aspect the invention provides the use of a zinc salt-containing oral care composition for preventing or reducing acidification within dental plaque in periods between brushing with said oral care composition.

In a third aspect the invention provides the use of a zinc salt in the manufacture of an oral care composition for preventing or reducing the dissolution of enamel caused by the acidification within dental plaque following the consumption of fermentable carbohydrate-containing comestibles.

Typically, the comestible will be any foodstuff which comprises some degree of fermentable carbohydrate, e.g. sweets/candies, soft drinks, especially the carbonated soft drinks, cakes, etc. and also causes a reduction of the pH within dental plaque by at least 0.5 pH units, more preferably at least 1.0 pH units, especially at least 1.5 and most preferably at least 2.0 pH units. This acidification remaining until around 30 min, preferably 60 min and most preferably around just 75 min from ingestion. These limits can be measured using pH touch electrodes or by harvesting plaque.

The acidification of dental plaque leads to the demineralisation of the enamel in the microenvironment of dental plaque. This is in contrast to the general pH of the oral cavity which tends to increase after consumption of various foods, acidic or otherwise because of the increased release of saliva. Typically, a pH of around 5.5 is thought to be the limit below which demineralisation starts to rapidly outperform remineralisation.

Preferably, the oral care composition comprises a zinc salt at from 0.01 to 5% by weight of the composition. Most preferably, the zinc salt is present at from 1.0 to 2.5% by weight and especially at 2% by weight of the composition.

Preferably, the zinc salt is zinc citrate, zinc sulphate, zinc gluconate, zinc acetate, and zinc chloride to name a few. Most preferably, it is zinc citrate.

Preferably, the reduction of 'pH drop' within the dental plaque effected by the zinc-containing oral care composition is a reduction of 20%, more preferably, 50% and most preferably 80% of the pH drop associated with the given comestible.

Where the effect is prevention of acidification it can also include anything up to a reduction to 5% what it would have been had no zinc-containing oral care composition have been administered.

The oral care composition is typically administered from 1 to 3 times in a 24 hour period. Preferably, it is administered twice a day: once at the start of the day and once at the end of the day. Should a further administration be required it is preferable that it occurs around midday. It is also preferable that administration occurs before mealtimes or snacking such that the prevention/reduction of acidification within dental plaque can occur once the food has been consumed. Even with just two administrations a day the effect can prevent or reduce acidification within the dental plaque caused by meals, snacks and drinks such as soft drinks and sugared tea/coffee for the remainder of the day.

The oral composition according to the invention may comprise further ingredients which are common in the art, such as:
antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
dental plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.
humectants such as glycerol, sorbitol, propyleneglycol, xylitol, lactitol etc.;
binders and thickeners such as sodium carboxymethylcellulose, xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate);
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral compositions may be in any form common in the art, e.g. toothpaste, gel, mousse, aerosol, gum, lozenge, powder, cream, etc. and may also be formulated into systems for use in dual-compartment type dispensers.

The zinc salt is incorporated into the oral care compositions in the usual manner well known to the person skilled in the art of oral care composition manufacture.

### EXAMPLE

The effect of a 2% Zinc citrate, 0.3% triclosan dentifrice on the plaque acid pH drop following consumption of a snackfood was measured by taking salivary samples from test subjects, centrifuging down the plaque and subjecting to a food challenge (Glucose) and then measuring the pH at various time intervals.

| Sample | A | B | C | D | E |
|---|---|---|---|---|---|
| 0 | 7.02 | 6.93 | 6.79 | 6.96 | 7.02 |
| 15 | 574 | 6.32 | 6.52 | 5.72 | 5.48 |
| 30 | 5.32 | 6.22 | 6.54 | 5.40 | 5.11 |
| 45 | 5.14 | 6.15 | 6.55 | 5.22 | 4.94 |
| 60 | 5.05 | 6.07 | 6.54 | 5.15 | 4.91 |
| 90 | 4.98 | 5.94 | 6.57 | 5.01 | 4.83 |

| | | | | | |
|---|---|---|---|---|---|
| A: Toothpaste comprising 1000 ppm fluoride | | | | | |
| B: Toothpaste comprising 0.75 % ZCT and 0.3% TCN | | | | | |
| C: Toothpaste comprising 2% ZCT and 0.3% TCN | | | | | |
| D: Toothpaste comprising 0.3% TCN and Gantrez | | | | | |
| E: Glucose (control) | | | | | |

The results clearly show that using a zinc containing toothpaste prior to consumption of a snackfood significantly reduces the drop in plaque pH associated with consumption of such.

## Claims

1. Use of a zinc salt-containing oral care composition for preventing or reducing acidification within dental plaque after consumption of a comestible, said comestible comprising a fermentable carbohydrate.

2. Use of a zinc salt-containing oral care composition for preventing or reducing acidification within dental plaque in periods between brushing with said oral care composition.

3. Use of a zinc salt in the manufacture of an oral care composition for preventing or reducing the dissolution of enamel caused by the acidification within dental plaque following the consumption of fermentable carbohydrate-containing comestibles.

4. Use according to any preceding claim, wherein said oral care composition comprises from 0.01 to 5% by weight zinc salt.

5. Use according to any preceding claim, wherein said oral care composition comprises zinc citrate.
